Europäisches Patentamt

European Patent Office

Office européen des brevets

(11) Numéro de publication: **0 364 310**
**A1**

(12) **DEMANDE DE BREVET EUROPEEN**

(21) Numéro de dépôt: **89401437.2**

(22) Date de dépôt: **25.05.89**

(51) Int. Cl.5: **B01D 53/36 , C07C 51/487**

Le titre de l'invention a été modifié (Directives relatives à l'examen pratiqué à l'OEB, A-III, 7.3)

(30) Priorité: **26.05.88 FR 8807015**

(43) Date de publication de la demande:
**18.04.90 Bulletin 90/16**

(84) Etats contractants désignés:
**BE DE ES FR GB IT NL**

(71) Demandeur: **Société Anonyme dite:**
**NORSOLOR**
**Tour Aurore, Place des Reflets**
**F-92080 Paris la Défense 2, Cedex 5(FR)**

(72) Inventeur: **Hazan, Charles**
**15, Passage Trubert-Bellier**
**F-75013 Paris(FR)**
Inventeur: **Esch, Marc**
**23, rue Goethe**
**F-57800 Freyming Merlebach(FR)**

(74) Mandataire: **Chaillot, Geneviève**
**Cabinet CHAILLOT 21, avenue Louise de**
**Bettignies**
**F-92700 Colombes(FR)**

(54) **Procédé de purification catalytique d'effluents aqueux contenant de l'acide acétique.**

(57) Pour purifier un effluent aqueux contenant de l'acide acétique comme sous-produit indésirable, il est proposé de mettre en contact un mélange d'alimentation à l'état de vapeur, composé dudit effluent à purifier et d'$O_2$, avec un catalyseur d'oxydation en $CO_2$ et $H_2O$ de l'acide acétique contenu dans ledit effluent, ledit catalyseur étant choisi parmi Pt, Rh et leurs mélanges, et les mélanges Bi-Cu.

Dans le cas où l'effluent est celui d'une réaction d'oxydation catalytique (par exemple, oxydéshydrogénation de l'acide isobutyrique en méthacrylate de méthyle) ayant nécessité de la vapeur d'eau de dilution, ladite réaction ayant été suivie par une extraction liquide-liquide ayant permis de récupérer, en phase organique, le produit recherché, la phase aqueuse constituant alors l'effluent à purifier, on met en contact un mélange d'alimentation tel que défini ci-dessus avec un catalyseur d'oxydation en $CO_2$ et $H_2O$ de l'acide acétique contenu dans ledit effluent, l'effluent purifié étant recyclé dans le réacteur d'oxydation en tant que vapeur d'eau de dilution.

EP 0 364 310 A1

## PROCEDE DE PURIFICATION CATALYTIQUE D'EFFLUENTS AQUEUX

La présente invention concerne la purification d'effluents aqueux contenant de l'acide acétique comme sous-produit organique indésirable principal.

La présente invention trouve son application notamment dans le cas des oxydations catalytiques qui nécessitent de la vapeur d'eau de dilution et qui conduisent à un effluent aqueux contenant le produit recherché et des sous-produits, dont l'acide acétique, le cas échéant avec les substances de départ n'ayant pas réagi, cet effluent, éventuellement après condensation, étant soumis à une extraction liquide-liquide permettant d'extraire, dans la phase organique, le produit utile de réaction et certains sous-produits, la phase aqueuse résultante contenant de l'acide acétique.

A titre d'exemples d'oxydations catalytiques, on peut mentionner tous les procédés de fabrication d'acide méthacrylique par oxydation de l'acide isobutyrique, d'isobutène ou de tertio-butanol, de méthacroléine, ainsi que d'acide acrylique par oxydation du propylène ou d'acroléine, utilisant de la vapeur d'eau de dilution et une extraction liquide-liquide.

En particulier, on peut citer la réaction d'oxydéshydrogénation de l'acide isobutyrique, ou d'un équivalent fonctionnel de cet acide, laquelle s'effectue en phase gazeuse, à une température élevée, de l'ordre de 300 à 400°C, sur un catalyseur et en présence de vapeur d'eau de dilution, pour obtenir respectivement l'acide méthacrylique, ou le dérivé à insaturation $\alpha,\beta$-éthylénique correspondant. Les brevets français n° 2 497 795, 2 499 557 et 2 514 756 illustrent de telles réactions d'oxydéshydrogénation.

Les effluents de réaction contiennent, dans le cas précité, de l'acide isobutyrique, de l'acide méthacrylique, de l'acétone, de l'acide acétique, de l'oxyde de carbone, du gaz carbonique et, naturellement, l'eau de dilution et celle de réaction. Après avoir condensé le mélange, on effectue une extraction liquide-liquide. L'acétone ainsi que les acides méthacrylique et isobutyrique sont assez facilement extraits; en revanche, l'acide acétique est difficilement extrait et présente une forte tendance à rester dans l'eau. Même si le solvant organique en extrait une partie, lors de la distillation du solvant, l'acide acétique aura tendance à passer en tête avec le solvant et sera donc renvoyé à la colonne d'extraction, de telle sorte qu'en fin de compte, la seule sortie d'acide acétique se fera par la phase aqueuse.

Cette phase aqueuse, qui contient donc de l'acide acétique, ne doit pas, pour des raisons de pollution, être rejetée dans le milieu naturel. Il est souhaitable d'en recycler au moins une partie au réacteur d'oxydéshydrogénation, après vaporisation, sous la forme de vapeur d'eau de dilution.

Or, l'acide acétique s'oxyde difficilement dans les conditions de cette réaction d'oxydéshydrogénation et il traversera essentiellement le réacteur, sans endommager le catalyseur employé, mais sans subir de transformation. En l'absence d'un traitement approprié, l'acide acétique va donc s'accumuler dans la boucle réaction/extraction liquide-liquide, jusqu'à des concentrations telles qu'il sera pratiquement entièrement éliminé dans la purge d'eau correspondant à l'eau créée dans la réaction d'oxydéshydrogénation. A ce stade, le bénéfice au plan de la pollution dudit recyclage devient négligeable.

Les mêmes constatations peuvent être présentées pour les autres réactions d'oxydation catalytique définies ci-dessus.

Il a déjà été proposé, dans le traitement catalytique de gaz résiduaires provenant de la synthèse de produits intermédiaires organiques, d'effectuer l'oxydation de l'acide acétique sur un catalyseur contenant du palladium, à 250-390°C (CA, Vol 103, 1985, 200238r) ; l'oxydation de l'acide acétique sur des catalyseurs consistant en oxydes composites de cobalt-bismuth a également été décrite (CA, Vol 98, 1983, 7725x) ; enfin, dans CA, Vol 94, 1981, 52174v, est décrit le traitement de gaz résiduaires contenant, entre autres, de l'acide acétique, en provenance de la fabrication de l'acide téréphtalique, par combustion catalytique sur un catalyseur de $Pd-Al_2O_3$, en présence de H, à 200°C et sous 22 atmosphères.

La Société Déposante a maintenant découvert que la purification des effluents aqueux, contenant de l'acide acétique comme sous-produit organique indésirable principal, pouvait être réalisée par la destruction en $CO_2$ et $H_2O$ de l'acide acétique, par une oxydation catalytique de ce dernier en phase vapeur, sur des catalyseurs déterminés. La Société déposante a également découvert que, dans le cas précité des effluents d'oxydations catalytiques utilisant de la vapeur d'eau de dilution et une extraction liquide-liquide, la purification de l'effluent pouvait être réalisée par la destruction en $CO_2$ et $H_2O$ de l'acide acétique, par une oxydation catalytique de ce dernier en phase vapeur, notamment sur un catalyseur dit «d'incinération en phase gazeuse» (métaux nobles le cas échéant supportés sur alumine ou silice et mélanges Bi-Cu également le cas échéant supportés sur alumine ou silice), avec recyclage de l'effluent purifié dans la réaction d'oxydation.

La présente invention a donc d'abord pour objet un procédé de purification d'un effluent aqueux contenant de l'acide acétique comme sous-produit indésirable, caractérisé par le fait que l'on met en

2

contact un mélange d'alimentation à l'état de vapeur, composé dudit effluent à purifier et d'oxygène, avec un catalyseur d'oxydation en $CO_2$ et $H_2O$ de l'acide acétique contenu dans ledit effluent, ledit catalyseur étant choisi parmi le platine et le rhodium et leurs mélanges ainsi que parmi les mélanges Bi-Cu.

Ces catalyseurs sont donc choisis parmi le platine, le rhodium ou un mélange des deux, pouvant être supportés sur alumine ou silice, comme décrit par exemple dans le brevet PL n° 124 741, et les mélanges Bi-Cu, pouvant également être supportés sur alumine ou silice, comme cité dans le brevet RD 235 014.

La présente invention a également pour objet un procédé de purification de l'effluent aqueux, contenant de l'acide acétique comme sous-produit indésirable, d'une réaction d'oxydation catalytique ayant nécessité de la vapeur d'eau de dilution, ladite réaction ayant été suivie par une extraction liquide-liquide ayant permis de récupérer, en phase organique, le produit recherché, la phase aqueuse constituant alors l'effluent à purifier, caractérisé par le fait que l'on met en contact un mélange d'alimentation à l'état de vapeur, composé dudit effluent à purifier et d'oxygène, avec un catalyseur d'oxydation en $CO_2$ et $H_2O$ de l'acide acétique contenu dans ledit effluent, et par le fait que l'effluent purifié est recyclé dans le réacteur d'oxydation, en tant que vapeur d'eau de dilution.

L'effluent gazeux purifié est donc ici avantageusement recyclé dans le réacteur d'oxydation. On constate donc que ce procédé de purification n'est pas d'un coût énergétique élevé, car la chaleur nécessaire à la vaporisation et à la surchauffe dans cette purification doit être fournie de toute façon pour le recyclage au réacteur d'oxydation.

Conformément à un mode préféré de réalisation des procédés selon l'invention, le rapport molaire de l'oxygène à l'acide acétique dans le mélange d'alimentation mis en contact avec le catalyseur est compris notamment entre 2 à 15 moles d'oxygène pour 1 mole d'acide acétique. Par ailleurs, l'oxygène utilisé pour la réaction peut être fourni sous forme d'air.

La réaction de purification selon l'invention est conduite à une température comprise notamment entre 300 et 600°C, de préférence, entre 325°C et 475°C, et sous une pression comprise notamment entre 0,1 et 10 bars (pression absolue), ladite pression étant, de préférence, voisine de la pression atmosphérique (1 bar ± 10%).

Le temps de contact du mélange d'alimentation avec le catalyseur peut varier, dans le procédé de l'invention, selon la température, la pression, le catalyseur et le mélange d'alimentation ; cependant, il doit être suffisant pour détruire, de façon substantielle, l'acide acétique en $CO_2$ et $H_2O$. En général, le temps de contact est compris notamment entre 0,01 et 10 secondes, de préférence, entre 0,1 et 3 secondes. Le temps de contact, ou durée de réaction, est défini comme le rapport du volume du catalyseur au débit de gaz d'alimentation en volume par seconde (mesuré dans les conditions standard). Le volume du catalyseur est le volume apparent occupé par le catalyseur dans le réacteur, mesuré à la température ambiante. (Le volume apparent du "catalyseur" comprend, non seulement le composé actif adsorbé à la surface, mais également le support du catalyseur).

Les exemples suivants illustrent la présente invention sans toutefois en limiter la portée.

## Exemple 1

On se propose de purifier, en éliminant l'acide acétique qu'elle contient, une solution aqueuse provenant d'une extraction liquide-liquide consécutive à une réaction d'oxydéshydrogénation de l'acide isobutyrique, cette solution aqueuse contenant 500 ppm d'acide acétique indésirable.

A cet effet, on mélange cette solution à raison de 125 ml/h, à un débit d'air égal à 1,66 Nl/h. On effectue la vaporisation de ce mélange et on le surchauffe pour le porter à une température de 370°C, puis on le fait passer, dans un réacteur tubulaire placé dans un bain de sel fondu maintenu à cette température, sur 0,043 litre d'un catalyseur d'incinération à base de Pt et Rh, préparé conformément au mode opératoire indiqué dans le brevet PL n° 124 741, avec un temps de séjour d'1 seconde.

On observe que 84% de l'acide acétique sont oxydés en $CO_2$ et $H_2O$.

L'effluent de cette réaction de purification est apte à servir de vapeur d'eau de dilution pour le catalyseur d'oxydéshydrogénation de l'acide isobutyrique.

## Exemple 2

On se propose de purifier une solution aqueuse provenant d'une extraction liquide-liquide consécutive à une réaction d'oxydéshydrogénation de l'acide isobutyrique, contenant 1000 ppm d'acide acétique.

A cet effet, on mélange cette solution aqueuse, à raison de 620 ml/h, à un débit d'air de 8,2 Nl/h. On

3

EP 0 364 310 A1

vaporise le mélange et on le surchauffe pour le porter à une température T, avant de le faire passer dans le réacteur de l'Exemple 1, sur la même quantité du même catalyseur, mais avec un temps de séjour de 0,2 seconde.

On a conduit plusieurs réactions de purification en faisant varier la température T. Les taux de destruction de l'acide acétique en $CO_2$ que l'on a obtenus à chaque fois sont rapportés dans le tableau suivant :

| Température de réaction T | Taux de destruction en $CO_2$ |
|---|---|
| 325° C | 84,2% |
| 375° C | 90,6% |
| 400° C | 90,5% |

**Revendications**

1 - Procédé de purification d'un effluent aqueux contenant de l'acide acétique comme sous-produit indésirable, caractérisé par le fait que l'on met en contact un mélange d'alimentation à l'état de vapeur, composé dudit effluent à purifier et d'oxygène, avec un catalyseur d'oxydation en $CO_2$ et $H_2O$ de l'acide acétique contenu dans ledit effluent, ledit catalyseur étant choisi parmi le platine et le rhodium et leurs mélanges, ainsi que parmi les mélanges Bi-Cu.

2 - Procédé selon la revendication 1, caractérisé par le fait que le catalyseur utilisé est supporté sur alumine ou silice.

3 - Procédé de purification de l'effluent aqueux, contenant de l'acide acétique comme sous-produit indésirable, d'une réaction d'oxydation catalytique ayant nécessité de la vapeur d'eau de dilution, ladite réaction ayant été suivie par une extraction liquide-liquide ayant permis de récupérer, en phase organique, le produit recherché, la phase aqueuse constituant alors l'effluent à purifier, caractérisé par le fait que l'on met en contact un mélange d'alimentation à l'état de vapeur, composé dudit effluent à purifier et d'oxygène, avec un catalyseur d'oxydation en $CO_2$ et $H_2O$ de l'acide acétique contenu dans ledit effluent, et par le fait que l'effluent purifié est recyclé dans le réacteur d'oxydation, en tant que vapeur d'eau de dilution.

4 - Procédé selon l'une des revendications 1 à 3, caractérisé par le fait que le rapport molaire de l'oxygène à l'acide acétique dans le mélange d'alimentation mis en contact avec le catalyseur est compris entre 2 et 15 moles d'oxygène pour 1 mole d'acide acétique.

5 - Procédé selon l'une des revendications 1 à 4, caractérisé par le fait que l'on conduit la réaction de purification à une température comprise entre 300 et 600° C.

6 - Procédé selon l'une des revendications 1 à 5, caractérisé par le fait que l'on conduit la réaction de purification sous une pression absolue comprise entre 0,1 et 10 bars.

7 - Procédé selon l'une des revendications 1 à 6, caractérisé par le fait que le temps de contact du mélange d'alimentation avec le catalyseur est compris entre 0,01 et 10 secondes.

8 - Procédé selon l'une des revendications 1 à 7, caractérisé par le fait que l'oxygène est fourni sous forme d'air.

9 - Procédé selon l'une des revendications 3 à 8, caractérisé par le fait que l'effluent à purifier est un effluent d'une réaction d'oxydéshydrogénation de l'acide isobutyrique en méthacrylate de méthyle.

4

# RAPPORT DE RECHERCHE EUROPEENNE

Office européen des brevets

Numero de la demande

EP 89 40 1437

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | CHEMICAL ABSTRACTS, vol. 97, 1982, page 335, résumé no. 132694s, Columbus, Ohio, US; T.M. DOBROMYSLOVA et al.: "Catalytic cleaning of an oxygen-containing gas flow with the removal of organic impurities under pressure" * Résumé * --- | 1-2 | B 01 D 53/36 C 07 C 51/487 |
| A | CHEMICAL ABSTRACTS, vol. 103, 1985, page 274, résumé no. 200238r, Columbus, Ohio, US; G.I. SOLOV'EV et al.: "Kinetics of exhaustive oxidation of acetic acid on a palladium-containing catalyst", & KHIM. TEKHNOL. (KIEV) 1985, (3), 54-6 * Résumé * --- | 1-2 | |
| A | CHEMICAL ABSTRACTS, vol. 98, 1983, page 196, résumé no. 7725x, Columbus, Ohio, US; S. IMAMURA et al.: "Oxidation of acetic acid on cobalt-bismuth composite oxide catalysts", & J. CATAL. 1982, 78(1), 217-24 * Résumé * --- | 1-2 | |
| A | CHEMICAL ABSTRACTS, vol. 94, 1981, page 316, résumé no. 52174v, Columbus, Ohio, US; & JP-A-80 99 517 (HITACHI LTD) 29-07-1980 * Résumé * --- | 1-2 | |
| A | FR-A-2 160 582 (JOHNSON MATTHEY & CO.) --- | | |
| A | US-A-4 277 453 (J. AIKEN) --- -/- | | |

**DOMAINES TECHNIQUES RECHERCHES (Int. Cl.4)**

B 01 D
B 01 J
C 07 C

Le présent rapport a été établi pour toutes les revendications

| Lieu de la recherche | Date d'achèvement de la recherche | Examinateur |
|---|---|---|
| LA HAYE | 21-08-1989 | KANOLDT W.W. |

EPO FORM 1503 03.82 (P0402)

Office européen
des brevets

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 246 031 (ATOMIC ENERGY OF CANADA LTD) --- | | |
| A | EP-A-0 075 124 (DEGUSSA) --- | | |
| A | DE-A-2 828 034 (GMC) --- | | |
| A | FR-A-2 376 686 (SHELL) --- | | |
| A,D | PL-A- 124 741 (J. STRASZKO) ----- | | |

DOMAINES TECHNIQUES RECHERCHES (Int. Cl.4)

Le présent rapport a été établi pour toutes les revendications

| Lieu de la recherche | Date d'achèvement de la recherche | Examinateur |
|---|---|---|
| LA HAYE | 21-08-1989 | KANOLDT W.W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)